# EUROPÄISCHE PATENTSCHRIFT

(11) **EP 0 930 934 B1**
(45) Veröffentlichungstag und Bekanntmachung des Hinweises auf die Patenterteilung: **30.05.2001**
(21) Anmeldenummer: 97910358.7
(22) Anmeldetag: 25.09.1997
(51) Int. Cl.: B01J 2/00, C08J 3/12, B01J 20/26

(54) **VERFAHREN ZUR VERGRÖSSERUNG DER OBERFLÄCHE VON PARTIKELN**
PROCESS FOR ENLARGING THE SURFACE OF PARTICLES
PROCEDE POUR AGRANDIR LA SURFACE DE PARTICULES

(30) Priorität: 26.09.1996 DE 19639491
(43) Veröffentlichungstag der Anmeldung: 28.07.1999
(73) Patentinhaber: FRAUNHOFER-GESELLSCHAFT ZUR FÖRDERUNG DER ANGEWANDTEN FORSCHUNG E.V., 80636 München (DE)
(72) Erfinder: EYERER, Peter, D-76228 Stupferich (DE); ELSNER, Peter, D-76327 Pfinztal (DE); EMMERICH, Rudolf, D-76646 Bruchsal (DE)
(86) Internationale Anmeldenummer: EP9705263
(87) Internationale Veröffentlichungsnummer: WO9813132

(56) Entgegenhaltungen:
- EP-A- 0 085 014
- EP-A- 0 514 775
- EP-A- 0 695 763
- DE-A- 2 327 956
- US-A- 4 513 513
- DATABASE WPI Section Ch, Week 8138 Derwent Publications Ltd., London, GB; Class B07, AN 81-69134D XP002055124 & JP 56 035 928 B (TOA YAKUHIN KOGYO KK) , 20.August 1981

## Beschreibung

Die Erfindung betrifft ein Verfahren zur Zerkleinerung von Polymerpartikeln, die zumindest in begrenztem Umfang Flüssigkeiten absorbieren, wobei die Polymerpartikel einer Flüssigkeit oder einer diese enthaltenden feuchten Atmosphäre solange ausgesetzt werden, bis die Flüssigkeit zumindest in den oberflächennahen Bereich der Polymerpartikel penetriert ist.

Das mechanische Zerkleinern von Polymerpartikeln der vorgenannten Art erfordert in der Regel einen erheblichen apparativen Aufwand und Energieeinsatz und ist somit zeit- und kostenaufwendig. Zudem lassen sich nur solche Partikel mechanisch zerkleinern, die eine gewisse Härte und damit Sprödigkeit besitzen.

Der EP-A-0 085 014 ist ein Verfahren zur Zerkleinerung elastischer thermoplastischer Polymerpartikel, wie Polyethersulfone, Polycarbonate oder Polysulfone, entnehmbar. Bei diesem Verfahren wird ein als "Streß-Spalt-Promotor" bezeichnetes Lösungsmittel, z.B. eine Acetonlösung, in die Oberfläche der Polymerpartikel diffundiert, wobei die Struktur der Partikel entlang der Diffusionspfade geschwächt wird. Anschließend werden die derart behandelten Partikel einer äußeren mechanischen Kraft, z.B. einer Kugelmühle, unterworfen, wobei die groben Partikel entlang der Diffusionspfade als Schwächungslinien aufbrechen und kleinere Partikel erhalten werden. Zur mechanischen Zerkleinerung ist folglich ein ähnlich hoher, mit einem hohen Energieeinsatz verbundener apparativer Aufwand erforderlich.

Die DE-A-2 327 956 beschreibt ein Verfahren zur Herstellung wasserlösliche, hydratisierte Salze enthaltender Granulaten, insbesondere granulierten Wasch- und Reinigungsmitteln. Um ein Granulat zu erhalten, welches in Schüttungen nicht zusammenbackt und keine Hydratisationswärme erzeugt, wird von einem Rohgranulat ausgegangen, das eine zur Hydratisierung unter Bildung eines festen, trocken erscheinenden Produktes geeignete Komponente (z.B. Salze polymerer Phosphrsäure, Alkalicarbonate etc.) sowie Wasser enthält, wobei das Wasser nicht nur in die Granulate eindiffundiert, sondern chemisorbiert wird, indem es eine Änderung des Kristallwassergehaltes der in den Granulaten zwingend enthaltenen wasserlöslichen hydratisierten Salzen bewirkt. Dieses Rohgranulat wird in noch nicht völlig verfestigtem, feucht erscheinendem Zustand durch Mikrowellenbestrahlung aufgebläht, bzw. verfestigt. Eine Zerkleinerung der Granulate wird nicht erwähnt.

Derwent-Abtract 81-69134D der JP 56 035 928 B offenbart ein Verfahren zur Herstellung von Granulaten aus pulverförmigen Materialien, wie Bariumsulfat, Aluminiumsiliziumoxid, Instantkaffee oder Milchpulver, indem das pulverförmige Material mit einer z.B. in Wasser, Ethanol, oder chlorierten Kohlenwasserstoffen gelösten, unter Hitze gaserzeugenden Substanz, wie Natriumhydrogencarbonat oder Hefe, als Gerüstsubstanz versetzt und mit Mikrowellenenergie erhitzt wird. Ziel ist die Herstellung eines im Vergleich mit dem pulverförmigen Ausgangsmaterial porösen Granulates für Arzneimittel, Nahrungsmittel etc. Das pulverförmige Ausgangsmaterial soll mit so viel Lösung versetzt werden, daß es in Mikrowellenkammern eingedüst werden kann. Die Granulierung selbst erfolgt mittels herkömmlicher Methoden, wobei die Mikrowellenenergie ausschließlich zum Austreiben der gaserzeugenden Substanz verwendet wird. Eine Zerkleinerung der Granulate wird auch hier nicht erwähnt.

Der Erfindung liegt die Aufgabe zugrunde, ein einfaches und kostengünstiges Verfahren vorzuschlagen, mit dessen Hilfe es gelingt, Polymerpartikel, die zumindest in begrenztem Umfang Flüssigkeiten absorbieren, ohne Anwendung mechanischer Kräfte zu zerkleinern.

Diese Aufgabe wird bei einem Verfahren gemäß dem Oberbegriff des Anspruchs 1 erfindungsgemäß dadurch gelöst, daß die flüssigkeitshaltigen Polymerpartikel mit Mikrowellen bis zum schlagartigen Verdampfen der penetrierten Flüssigkeit bestrahlt werden, so daß die damit einhergehende spontane Druckerhöhung im Molekülverbund die vorhandene Struktur der Polymerpartikel zur Gänze oder nur an deren Oberfläche sprengt.

Die Erfindung nutzt die Absorptionsfähigkeit -gegebenenfalls auch im intermolekularen Bereich- einer Vielzahl von Polymeren aus, um in einer vorbereitenden Maßnahme in den Polymerpartikeln eine Flüssigkeit anzureichern und diese dann durch Mikrowellenenergie von innen heraus schlagartig zu verdampfen. Durch die damit einhergehende spontane Druckerhöhung im Partikelgefüge, insbesondere auch im Molekülverbund, wird die vorhandene Struktur der Polymerpartikel zur Gänze oder nur an deren Oberfläche gesprengt. Als Flüssigkeit kommt vornehmlich Wasser, aber auch jede andere Flüssigkeit in Frage, insbesondere auch solche niedrigerer Viskosität und/oder niedrigeren Siedepunktes in Frage. Für die Durchführung des Verfahrens werden je nach Art der Polymerpartikel sowie je nach dem gewünschten Zerkleinerungseffekt Mikrowellen im Leistungsbereich von 10 W bis 50 kW eingesetzt. Die Bestrahlungsdauer kann zwischen 1 s und wenigen Minuten betragen.

Durch die Verweilzeit der Polymerpartikel in der Flüssigkeit bzw. in der feuchten Atmosphäre läßt sich bei einem gegebenen Penetrationskoeffizienten die Eindringtiefe der Flüssigkeit in etwa vorausbestimmen.

In einer Ausführungsform des Verfahrens werden die Polymerpartikel solange der Flüssigkeit bzw. der diese enthaltenden feuchten Atmosphäre ausgesetzt, daß die Flüssigkeit bis in den Kern der Partikel penetriert. Werden solche Polymerpartikel der Bestrahlung mit Mikrowelle ausgesetzt, so werden sie durch die Dampfbildung zur Gänze gesprengt und es entstehen kleinere Bruchstücke, die gegebenenfalls eine große unregelmäßige Oberfläche aufweisen. Dieses Verfahren eignet sich insbesondere z.B. zur Gewinnung eines feinkörnigen bis pulvrigen Haufwerks aus groben Polymerpartikeln. Das Verfahren ist besonders dort geeignet, wo sich die Polymerpartikeln durch herkömmliche mechanische oder chemische Methoden nicht weiter zerkleinern lassen. Dies gilt für harte ebenso wie für weiche oder mehr oder minder plastische Polymerpartikel.

So sind eine Reihe von Polymeren bekannt, die Feuchtigkeit bzw. Flüssigkeiten, insbesondere auch Wasser, in mehr oder minder großem Umfang aufnehmen. Hierzu zählen beispielsweise einige Thermoplaste, z.B. Acrylate, Polycarbonate und Polyamide. Bei diesen Polymeren zeigt sich die Flüssigkeitsaufnahme in der Regel in einer Verminderung der Oberflächenhärte.

Daneben gibt es Polymere, die bewußt im Hinblick auf eine hohe Absorptionsfähigkeit von Flüssigkeiten entwickelt wurden und die bei der Flüssigkeitsaufnahme in einen gelartigen Zustand übergehen. Diese Polymere werden als Super Absorbent Polymers (SAP) bezeichnet. Solche Super Absorbent Polymers (SAP) werden in Form von Perlen, beispielsweise zur Feuchtigkeitsabsorption im Humanbereich bei Windeln, Einlagen etc. verwendet. Diese SAP sind vergleichsweise teuer. Das Kornspektrum ist durch den Herstellungsprozeß weitgehend vorgegeben. Dies gilt auch für ihre vergleichsweise glatte Oberfläche. Durch das erfindungsgemäße Verfahren können auch nur Oberflächenbereiche abgesprengt werden. Dabei ergibt sich nicht nur durch die Haufwerkbildung eine Oberflächenvergrößerung, sondern wird ein zerklüftetes Einzelkorn mit entsprechend vergrößerter Oberfläche erhalten. Damit läßt sich die Absorptionsgeschwindigkeit erhöhen, so daß die SAP entweder wirkungsvoller oder in geringerer Menge eingesetzt werden können. Praktische Versuche haben gezeigt, daß in diesem Anwendungsfall Mikrowellenleistungen zwischen 100 W bis 5 kW bei einer Bestrahlungsdauer zwischen 1 s und 5 min und einer penetrierten Flüssigkeitsmenge von 1 bis 100 Mass.% zu einer Zerkleinerung führen. Dabei ist lediglich darauf zu achten, daß die bei der Bestrahlung auftretende Temperaturerhöhung innerhalb der Partikel nicht zu einer unerwünschten Veränderung der Molekularstruktur führt, was durch die Leistung und Dauer der Bestrahlung, wie auch durch den Gehalt an penetrierter Flüssigkeit steuerbar ist.

## Patentansprüche

1. Verfahren zur Zerkleinerung von Polymerpartikeln, die zumindest in begrenztem Umfang Flüssigkeiten absorbieren, wobei die Polymerpartikel einer Flüssigkeit oder einer diese enhaltenden feuchten Atmosphäre solange ausgesetzt werden, bis die Flüssigkeit zumindest in den oberflächennahen Bereich der Polymerpartikel penetriert ist, dadurch gekennzeichnet, daß die flüssigkeitshaltigen Polymerpartikel mit Mikrowellen bis zum schlagartigen Verdampfen der penetrierten Flüssigkeit bestrahlt werden, so daß die damit einhergehende spontane Druckerhöhung im Molekülverbund die vorhandene Struktur der Polymerpartikel zur Gänze oder nur an deren Oberfläche sprengt.

2. Verfahren nach Anspruch 1, dadurch gekennzeichnet, daß die Polymerpartikel der Flüssigkeit oder einer diese enthaltenden feuchten Atmosphäre solange ausgesetzt werden, daß die Flüssigkeit nur in den oberflächennahen Bereich eindringt.

3. Verfahren nach Anspruch 1, dadurch gekennzeichnet, daß die Polymerpartikel der Flüssigkeit oder einer diese enthaltenden feuchten Atmosphäre solange ausgesetzt werden, bis diese in den Kern der Polymerpartikel penetriert ist.

4. Verfahren nach Anspruch 2 zur Herstellung von Polymerpartikeln mit aufgerauhter Oberfläche.

## Claims

1. Method for reducing the size of polymer particles, which absorb fluids at least to a limited extent, the polymer particles being exposed long enough to a fluid or to a moist atmosphere which contains this fluid until the fluid has penetrated at least into the near-surface region of the polymer particles, characterised in that the polymer particles which contain fluid are irradiated with microwaves until the abrupt evaporation of the penetrated fluid, so that the thereby accompanying spontaneous pressure increase in the molecular bond ruptures the existing structure of the polymer particles entirely or only on their surface.

2. Method according to claim 1, characterised in that the polymer particles are exposed long enough to the fluid or a moist atmosphere which contains this fluid, that the fluid penetrates only into the near-surface region.

3. Method according to claim 1, characterised in that that the polymer particles are exposed long enough to the fluid or to a moist atmosphere which contains this fluid, until said fluid has penetrated into the core of the polymer particles.

4. Method according to claim 2 for producing polymer particles with a roughened surface.

## Revendications

1. Procédé de réduction de particules de polymère qui absorbent au moins dans une mesure limitée, des liquides, dans lequel les particules de polymère sont exposés à un liquide ou à une atmosphère contenant celui-ci, jusqu'à ce que le liquide pénètre au moins dans la zone à proximité de la surface des particules de polymère,
caractérisé en ce que
les particules de polymères contenant le liquide sont irradiées avec des micro-ondes jusqu'à évaporation soudaine du liquide qui a pénétré, de sorte que l'augmentation de pression spontanée progressive avec celle-ci dans la liaison moléculaire fasse éclater la structure présente des particules de polymère dans son intégralité ou seulement à leur surface.

2. Procédé selon la revendication 1,
caractérisé en ce que
les particules de polymère sont exposées au liquide ou à une atmosphère humide contenant celui-ci jusqu'à ce que le liquide pénètre seulement dans la zone à proximité de la surface.

3. Procédé selon la revendication 1,
caractérisé en ce que
les particules de polymères sont exposées au liquide ou à une atmosphère humide le contenant jusqu'à ce que celui-ci ait pénétré dans le noyau des particules de polymère.

4. Procédé selon la revendication 2 pour la production de particules de polymère ayant des surfaces rendues rugueuses.
